# EUROPEAN PATENT SPECIFICATION

(11) **EP 1 318 723 B1**
(45) Date of publication and mention of the grant of the patent: **18.11.2009**
(21) Application number: 01985229.2
(22) Date of filing: 17.09.2001
(51) Int. Cl.: A23C 19/032, A23C 19/04, C12N 11/02, A23C 19/05, A23P 1/04

(54) **TARGETING OF ACTIVE COMPOUNDS TO CURD DURING CHEESE MAKING**
STEUERUNG VON AKTIVEN SUBSTANZEN ZUM BRUCH WÄHREND DER KÄSEHERSTELLUNG
CIBLAGE DE COMPOSES ACTIFS SUR LE CAILLE LORS DE LA FABRICATION DE FROMAGE

(30) Priority: 19.09.2000 EP 00203272; 14.12.2000 EP 00204533
(43) Date of publication of application: 18.06.2003
(73) Proprietor: DSM IP Assets B.V., 6411 TE Heerlen (NL)
(72) Inventor: HAAN, DE, Ben, Rudolf, 2272 EN VOORBURG (NL); ROOS, DE, Andre, Leonardus, 2614 TB Delft (NL)
(74) Representative: Matulewicz, Emil Rudolf Antonius
(86) International application number: PCT/EP2001/010972
(87) International publication number: WO 2002/023998

(56) References cited:
- EP-A- 0 188 067
- EP-A- 0 750 854
- US-A- 3 746 621
- US-A- 5 686 385
- US-A- 6 068 705
- YOSHIMARU T ET AL: "PREPARATION OF MICROCAPSULATED ENZYMES FOR LOWERING THE ALLERGENIC ACTIVITY OF FOODS" JOURNAL OF AGRICULTURAL AND FOOD CHEMISTRY,US,AMERICAN CHEMICAL SOCIETY. WASHINGTON, vol. 45, no. 10, 1 October 1997 (1997-10-01), pages 4178-4182, XP000701990 ISSN: 0021-8561
- KAILASAPATHYK ET AL: "STUDIES ON ENCAPSULATING ENZYMES TO ACCELERATE CHEESE RIPENING" AUSTRALIAN JOURNAL OF DAIRY TECHNOLOGY,DAIRY INDUSTRY ASSN AUSTRALIA, HIGHETT VIC,AU, vol. 53, no. 2, 1 June 1998 (1998-06-01), page 125 XP000772499 ISSN: 0004-9433
- C. PEROLS: "The potential of enzyme entrapment in konjac cold-melting beads" ENZYME AND MICROBIOLOGY TECHNOLOGY, vol. 20, no. 1, 1997, pages 57-60, XP000982955
- WRIGHT P M: "MICROENCAPSULATION OF LYSOZYME TO ACCELERATE AND MODIFY THE RIPENING OF CHEADDAR CHEESE" CULTURED DAIRY PRODUCTS JOURNAL,XX,XX, 1 May 1990 (1990-05-01), pages 11-14, XP000646533
- B. LAW: "Microencapsulated enzymes for cheese technology" NORTH EUROPEAN FOOD AND DAIRY JOURNAL, vol. 53, no. 6, 1987, pages 194-199, XP000983091
- JANSSEN A G W: "MOGELIJK SOELAAS VOOR VERSNELDE KAASRIJPING" VOEDINGSMIDDELEN TECHNOLOGIE,NOORDERVLIET B.V. ZEIST,NL, vol. 32, no. 8, 8 April 1999 (1999-04-08), page 32 XP000822923 ISSN: 0042-7934

## Description

### Field of the invention

The present invention relates to methods for the entrapment of an active compound in the curd during cheese making.

### Background of the invention

Traditional cheese manufacturing processes involve coagulating milk to form solid curds and liquid whey. Typically, the milk is enzymatically coagulated using rennet. The rennet can be of animal origin, but nowadays bacterial rennets are also used. The activity of the rennet leads to the hydrolysis of the Phe₁₀₅-Met₁₀₆ bond of the κ-casein. As κ-casein is the principal factor stabilising the casein micelles, its hydrolysis destabilises the micelles, which then coagulate in the presence of a critical Ca²⁺ concentration. For most cheese manufacturing processes the temperature at which the cheese milk coagulates is around 30°C. Following the coagulation of the cheese milk the curd particles are separated from the whey. The curd contains approximately 80% of the protein from the milk (caseins) and the whey the rest (whey protein). The curd is then salted and pressed to produce fresh cheese.

The fresh cheese is then aged (ripened) under the particular temperature conditions necessary to allow the desired taste and texture to develop. If the ageing or ripening period is short, the product is known as young cheese. Longer ageing periods produce mild, mature and extra mature cheese. As the cheese is aged for longer, ageing progresses more flavour develops, increasing the value of the resultant cheese. While "old" and "very old" cheeses command higher prices in the marketplace than younger cheeses, they are expensive to produce because of the substantial costs associated with storage and refrigeration during the lengthy ripening process. In many cases, ripening requires up to three to twelve months of storage at a temperature of about 10°C.

If ripening could be accelerated this would result in a reduction in the considerable costs associated with storing the cheese during the ripening process.

Many enzymes originated for example from the used starter culture, such as proteases, peptidases and lipases, are utilised as tools for adjusting the complex taste development of cheeses.

During the transition from liquid milk to the gel state, the caseins present separate from the whey fractions of the milk. Caseins make up approximately 80% of the milk proteins which collectively form the cheese curd. The remaining proteins are classified as whey proteins. In general the curd is separated from the whey fraction in about 30-50 minutes after the addition of the coagulant to the milk. A key problem in utilising enzymes in cheese production is targeting these enzymes to the curd in such a way that sufficient enzyme activity is retained within the cheese curd during gelation. Typically, enzymes are lost during cheese manufacture during the curd / whey separation. Therefore, there is a need for a means for retaining ripening enzymes within curd fractions during the production of the cheese.

Kailasapathy et al., Australian Journal of Dairy Technology, Vol. 53 (1998), page 125, XP000772499 describes studies on encapsulating enzymes to accelerate cheese ripening. As encapsulating materials food gums such as agar, alginate, gellan, kappa and iota-carrageenan and kappa-carrageenan-locust bean gum are disclosed.

C. Perols et al., Enzyme and Microbial Technology 20 (1997), page 57-60, XP-000982955 discloses the enzyme entrapment in konjac cold-melting gel beads to be used for controlled release for cheese ripening.

M. Wright et al.: Cultured Dairy Products Journal, (May 1990), page 11-14, XP-000646533 discloses microencapsulation of lysozyme to accelerate and modify the ripening of cheddar cheese.

B. Law et al.: North European Food and Dairy Journal, vol. 6/87 (1987), pages 194-199, XP-000983091 gives an overview of methods for micro-encapsulation of enzymes for cheese technology. Encapsulated lipases in formaldehyde-treated gelatin are mentioned.

A.G.W. Janssen: Voedingsmiddelen Technologie, nr. 8 (8 April 1999), page 32, XP000822923 discloses a liquid enzyme system for use in accelerated cheese ripening. It is disclosed that proteases are immobilized on larger, food grade particles. As particles soy oil droplets, caseins absorbed to soy oil droplets, and casein micelles were used.

U.S. Patent No. 4,927,644 describes a method for the entrapment of enzymes in cheese curd by producing enzyme aggregates without complexing or immobilising agents. The enzyme particles are insoluble in milk and when the curd is separated from the whey, the enzyme aggregates remain in the curd. However, as the enzyme aggregates consist essentially of insoluble enzyme after the separation of the curd and whey these enzymes are, at best, partly available for the cheese making process.

U.S. Patent 3,746,621 describes an enzyme containing microcapsule comprising an enzyme and particles of a finely divided swellable solid encapsulated in a binder substance. More particularly, it describes an enzyme-containing microcapsule which, in dry state, protects the enzyme but, when put in water, quickly break, down forming an aqueous solution of the enzyme. Examples of a binder solution are aqueous solutions of a water-soluble material, such as gelatin, casein, dextrin, dextran, or the like. Examples of swellable particles are silica, clay, starch, powdered dextran, gelatin, casein and the like.

### Summary of the invention

To solve the above-mentioned problems, the invention provides methods for the targeting of active compounds such as enzymes to cheese curd. The methods permit the release of the active compounds only after the separation of the curd and whey and moreover the active compound is homogeneously distributed throughout the curd.

Accordingly the present invention provides a matrix comprising:
- a hydrocolloid; and
- an enzyme in an amount from 0.01 to 20% w/w (based on dry matter of the matrix) ;
wherein the hydrocolloid is a protein, the matrix is in the form of particles and at least 50 % of the matrix particles have a diameter of from 1 to 50 µm, the matrix has from 80 to 99.9 % (w/w) dry matter content, and the enzyme is entrapped in the matrix.

The present invention further provides:
- a method for the production of a cheese which comprises the addition of a matrix of invention wherein the matrix comprises an enzyme and is added prior to the separation of the curds and whey.

### Detailed description of the invention

It has been surprisingly found that enzymes can be entrapped in a matrix, which is chosen or produced in such a way that it is homogeneously present in the curd. The majority of the enzymes will be released into the curd after the separation of the curd from the whey.

According to one embodiment of the invention the enzymes are entrapped in a matrix of hydrocolloid material. The matrix consists of proteins. Examples of suitable materials are gelatine, casein, whey, soybean protein, albumin. Casein and whey are the preferred food grade hydrocolloids for use in the invention, because they are already naturally present in cheese. Products derived from these hydrocolloids can also be used, such as for example sodium caseinate.

Also one or more of the above mentioned hydrocolloids may be used in combination with each other. In a preferred embodiment casein and whey are used. The enzyme is preferably homogeneously distributed throughout the matrix.

The enzyme is incorporated into the final cheese product because of the matrix. In general the enzyme will have a function during the ripening or storing process of the cheese. The enzyme may influence for example the structure, taste, storability, and ageing process of the cheese.

A homogenous distribution of the entrapped enzyme may give rise to some initial leakage. This can be reduced by drying the matrix. Accordingly, the matrix will be in a dry form containing from 80 to 99.9% (w/w) dry matter, depending on the hydrocolloid used as will be appreciated by the skilled person, preferably from 90 to 99.5%(w/w) and most preferably from 92 to 98% (w/w dry matter) when added to the milk.

In general from 0.01 to 5% (w/w) of matrix (containing the active compound, i.e. an enzyme) will be added to milk. Preferably from 0.1 to 2% w/w is added in normal cheese making. The matrix itself will contain from 0.01 to 20% w/w (based on dry matter) of the enzyme preferably from 0.05 to 10% and more preferably from 0.1 to 5% w/w.

Examples of enzymes, which are added as the active compound, are proteases, peptidases, amino transferases, lysases and lipases and combinations thereof. Preferably soluble enzymes are used.

The swell function of the hydrogel matrix is used to prevent the initial leakage of the active compounds. The enzymes are preferably present as soluble enzymes and therefore will only dissolve in the hydrogel matrix when the matrix is contacted with the milk. The dried hydrogel matrix will initially swell and only when enough water is absorbed will the enzymes dissolve and diffuse into the milk. In this way it is possible to entrap the enzymes long enough in the matrix, so that the enzymes are only substantially available after that the separation of the curd from the whey.

At least 40%, preferably at least 50%, more preferably at least 70% and even more preferably at least 90% (w/w) of the enzyme is retained in the curd until after the curd/whey separation step. The retainment or entrapment of the enzyme in the curd or cheese is preferably determined according to the procedure as described in Example 7. Because the matrix is in general intended for use in cheese making, the enzymes to be entrapped will advantageously have an effect in the cheese or the cheese making process and are in general food grade compounds.

It has also been found that the selection of the particle size of the matrix is an important aspect of the invention. Proper matrix particles must be large enough to be entrapped in the curd, for example greater than 1µm as measured by particle size Analysers by means of forward laser light scattering like the Malvern Mastersizer. Dried matrix particles of less than 50 µm are small enough to be present homogeneously in the curd without affecting the structure of the curd and resultant cheese. Consequently, 50% (w/w), preferably 70%, and more preferably 90% of the dried matrix particles of the invention have a size from 1 to 50 µm, preferably from 5 to 30µm. By particle size it is meant here the largest diameter of a particle.

The density of the swollen matrix has to be chosen in such a way that matrix is homogeneously distributed throughout the curd. Accordingly the specific gravity of the swollen matrix (in wet form) is preferably close to the density of milk. The density of the matrix can be adjusted, if necessary, by adding additional compounds. By incorporating for example fats or waxes into the matrix the density of the matrix can be decreased. These fatty compounds are preferably added to the matrix in dispersed form. To incorporate these fatty compounds in general emulsifiers will used. Preferably the emulsifier is identical to the matrix. For example sodium caseinate can be used for the matrix as well as emulsifier. Typically, for the matrix a high concentration caseinate will be used whereas the fatty materials will be emulsified in a lower caseinate solution. The concentration and the type of fat or wax can also be selected to slow the swelling process. In general the more wax or fat that is used, the slower the swelling process.

As explained above several mechanisms are available to control the release of the enzyme from the matrix at a selected or preferred time. The release of the enzyme can furthermore be actively influenced by adding compounds such as fats or waxes or by selecting the proper matrix/enzyme ratio. In general the higher this ratio, the lower the release rate of the enzyme.

According to one embodiment of the invention the enzyme can be entrapped in the matrix by introducing the enzyme into a soluble form of the matrix and subsequently changing the environment of the matrix, to cause the matrix to solidify. According to one embodiment a pH change is used to obtain solidification, for example lowering the pH of a sodium caseinate solution will coagulate the matrix.

Another way of retaining the enzyme within the matrix can be achieved by selecting hydrocolloids which are fluid at a higher temperature but which solidify at lower temperature, such as gelatine. This means enzyme immobilisation by temperature reduction is possible. Albumin can also be used. A temperature increase can be used to fixate the active compound in a solidified albumin matrix. In still another embodiment of the invention salt targeting is used. In this case, a matrix is chosen whereby salts are used to fixate the soluble hydrocolloids.

One of the preferred matrix systems is casein. Typically a highly concentrated sodium caseinate solution is mixed with the active compound. This aqueous solution preferably contains 10 to 40% w/w sodium caseinate, preferably 10 to 35% w/w, more preferably 15 to 30% w/w. Preferably the active compound is dissolved in water before it is added to the concentrated solution of sodium caseinate.

After mixing, acid is added in order to decrease the pH to approximately the isoelectric point to induce coagulation. As a result the active compound is completely entrapped in the coagulated casein. In case, where a low concentration sodium caseinate solution is used, synerase may occur and part of the activity of the active compound may be lost, therefore high concentrations of sodium caseinate are preferred.

Subsequently, the still wet coagulated matrix is preferably dried and milled to produce fine particles. Drying and milling processes known in the art can be used, such as for drying, vacuum drying and fluidised bed drying.

After drying and milling the casein matrix will have a particle size from 1-50 µm, more preferably from 5 to 30µm and even more preferably from 10 to 20µm.

In general a formulation suited for immobilising enzymes for use in the invention can be prepared in the following way.

First solution of a suitable matrix is mixed together with the enzyme.

The mixing can be done in any suitable mixer, for example with an electric dough mixer equipped with a butterfly whisk. The mixture solidifies while the mixing continues.

The obtained matrix particles contain the enzyme and are subsequently dried, for example, on a vacuum plate dryer.

The dried particles can be milled, for example with a centrifugal cutting mill.

The obtained dry powder can be stored at room temperature.

Another preferred matrix for use in the invention is whey protein. Heat denaturation will unfold such globular proteins into more or less random coils of peptide chains that on cooling will form aggregates via various types of interactions. These interactions may be of electrostatic, hydrophobic or covalent nature. The cross-linking interactions will entrap water molecules and a gel will be formed. A protein gel may be defined as a three dimensional matrix or network in which polymer-polymer interactions occur in an ordered manner resulting in the immobilization of large amounts of water by a small proportion of the protein.

Functionalities of the gel such as viscoelasticity and outward appearance (opalescence) are determined by process variables such as pH, ionic strength, valency of ions, concentration of constituents, extent of protein denaturation and time of reaction. The actual gelation will take place when the solution of the proteins has already cooled down. Furthermore the moment of gelation can be initiated by the addition of ions such as sodium or calcium.

It has been found that during the process of gelation of hydrocolloids, active proteins (such as enzymes) or carbohydrates can be entrapped in the gel cross-linking network. As gelation will take place at lower temperatures active proteins will not lose activity due to heat denaturation.

Further processing of such a whey protein based gel consisting of cutting the gel, drying and grinding will result in dried whey protein particles of defined size containing the entrapped active compound. In this way a food grade formulation of a bio-active hydrocolloid can be made with full retention of the activity of the active compound. During the cheese making process, due to the size of the particles, the active compound will be entrapped substantially in the curd and will not end up in the whey fraction or will only partly end up in the whey fraction.

The matrix of the present invention can be used in the production of hard or soft cheese such as cheddar, gouda, edam, etc.

### Measurement

This example describes the method that was used to analyze the amount of active amino-peptidase. Enzyme powder is dissolved in a 0.05 M phosphate buffer, pH = 7.2 at 20°C by suspending 1 g enzyme powder in 99 ml buffer solution. The obtained enzyme solution is accordingly diluted 10, 100 and 1000 fold to obtain the appropriate solution for the final enzyme activity analyses.

The obtained enzyme solution is analyzed by incubating 0.1 ml enzyme solution as such or diluted in a dilution range. The incubation (30°C) is done in a cuvet by mixing with 0.9 ml of a 260 ppm solution of L-phenylalanine-para-nitroanilide (purchased from Sigma P 4673) in 0.05 M phosphate buffer, pH = 7.2 against a blanc in time. The measurement was done at 30° C, during 4 minutes using an Uvicon 933 spectrophotometer. The activity was calculated from the delta extinction per minute using a molar extinction coefficient of 8800 ml/mol*cm.

### Measurement of enzyme activity present in the matrix

This example describes the method that was used to analyze the amount of active immobilized amino-peptidase.1g of the enzyme particles were suspended in 100 ml of a phosphate buffer (0.05M, pH=7.2, 20°C). The suspension time is varied to obtain information about the release time of the active enzyme from the particles.

The dissolved enzyme solution is isolated from the dispersion by pipetting directly or by centrifuging the dispersion and pipetting from the clear top layer.

The obtained enzyme solution is analyzed by incubating at 30°C, 0.1 ml enzyme solution as such and 0.1 ml samples from a dilution range of 10, 100 and 1000 fold to obtain the appropriate enzyme concentration. The incubation in the cuvet was done with 0.9 ml of a 260 ppm solution of L-phenylalanine-para-nitroanilide (purchased from Sigma P 4673) in 0.05 M phosphate buffer, pH = 7.2 against a blanc in time.

The measurement was done at 30° C, during 4 minutes using an Uvicon 933 spectrophotometer.

The activity was calculated from the delta extinction per minute using a molar extinction coefficient of 8800 ml/mol*cm.

### EXAMPLES

The following examples illustrate the invention.

### Comparative Example 1

The optimal concentration of matrix necessary to obtain a solidified matrix enzyme system without accompanying external moisture (synerase) during gelation was determined. This allowed enzyme loss during formulation to be minimised.

Sodium-caseinate, type Miprodan 30 (purchased MD Foods Ingredients Denmark) was dissolved in tap water at 20°C in concentrations of 10% (w/w), 15% (w/w), 20% (w/w) and 25% (w/w).

Solidification was obtained by heating 1000 g of dissolved protein up to 50° C to reduce the viscosity and accordingly solidifying the solution during stirring by adding a solution of 4M acetate buffer pH=4.0.

All concentrations of sodium casein are performed adequately but the best results were obtained with a concentration of at least 20% (w/w) sodium-caseinate.

### Example 2

The enzyme Accelerzyme™ AP 2000 (an amino-peptidase) was immobilized in sodium-caseinate type Miprodan 30.

250 g of sodium-caseinate was slowly added to 715 g of tap water at 20°C. The two were then stirred together with an electric dough mixer on stand 1, type N-50, (from Hobart) equipped with a butterfly whisk.

When the sodium-caseinate was completely dispersed into the water the mixture was heated to 50°C and mixed until the sodium-caseinate was completely dissolved.

10 g Accelerzyme AP 2000 (DSM-Gist, Delft, Holland) was dissolved in 25-g tap water at 50°C and was then added to the sodium-caseinate mixture while stirring.

To the stirred solution 30 g of Na-acetate buffer (4M, pH=4.0) was added slowly in 5 g portions and this solution was stirred until complete solidification of the solution occurred.

The obtained particles were collected and subsequently dried by spreading onto the drying plates of a vacuum plate dryer overnight at 30°C and 0.1 bar absolute pressure.

The obtained dried particles were then milled with a centrifugal cutting mill type ZM1, (from Retsch) with a throughput metal filter of 120 µm.

90 % of the counted particles had an average particle size of 10- 15 µm when measured with a Malvern Mastersizer S particle size analyser.

The obtained powder was collected and stored at room temperature until usage.

### Example 3

The enzyme Accelerzyme™ AP 2000 was immobilized in a gelated whey protein matrix. A Whey Protein Isolate (WPI) of type Bipro (Danisco International Inc.) was used.

10 g of WPI was dissolved overnight in 100 mL of distilled water milli-Q with slow stirring. The solution was extensively dialysed (molecular porous membrane m.w. cut off approx. 3.5000 Da - Spectra-Por) in 5 L milli- Q water to reduce ionic strength. The water was refreshed two times.

The solution was heated for 30 min to 85°C. After cooling to 30°C 664 mg of the Accelerzyme was added to 100 mL of the WPI solution. The pH of the solution was adjusted to pH = 10 with 0.1 N NaOH. 5% by volume of a CaCl2 (0.2 M) solution was added to the solution. At 30°C gelation occurred to give a clear gel. The gel was cut into small parts (about cc size) and deep frozen (- 20°C).

The gel was then vacuum dried for 48 hours and then ground in a mortar. The obtained powder was collected and stored at 4°C with further usage.

### Example 4

The suspension behaviour of the particles described in Examples 2 and 3 was evaluated.

In order to have a good visible interpretation of the suspension capacities of the obtained particles, the particles were introduced into a synthetic milk medium designed by Jenness & Koops (Jenness R. & Koops J. Neth Milk Dairy J. (1962)).

1 g of the powders obtained Examples 2 and 3 was added to 100 ml of the Jenness-Koops medium and mixed.

The suspension was observed for sedimentation, floating on the surface and suspension randomly in the solution over 30 minutes. No sedimentation or floating was observed.

### Example 5

The release of the enzyme from the particles obtained in Example 2 during hydration was studied.

The particles were suspended to conform to the method used in Example 4 and stirred gently for 3 days.

Samples were taken from the aqueous phase of the stirred suspension at various time points and subsequently measured for specific enzyme activity.

The results are presented in Table 1

**Table 1. Enzyme release during hydration of the dried enzyme powder**

| Sample time after hydration | Relative enzyme activity in the aqueous phase % |
|---|---|
| 30 minutes | 0 |
| 60 minutes | 3 |
| 90 minutes | 6 |
| 72 hours | 56 |

| | |
|---|---|
| Relative activity: relative to the enzyme activity before fixation in matrix. | |

### Example 6

The release of the enzyme from the particles obtained in Example 3 during hydration was studied.

The particles were suspended to conform to the method used in Example 4 and stirred gently for one hour.

Samples were taken from the aqueous phase of the stirred suspension at various time points and subsequently measured for specific enzyme activity. The results are presented in Table 2.

**Table 2.**

| Dissolving time (min) | Activity of Accelerzyme in supernatant (%) |
|---|---|
| 10 | 9.8 |
| 20 | 34.9 |
| 40 | 47.5 |
| 60 | 53.6 |

### Example 7

The enzyme powder of Example 2 was introduced into cheese.

The cheese was made according the following method (Shakeel-Ur-Rehm an, McSweenly, P.L.H. and Fox, 8.F., 1998. Protocol for the manufacture of miniature cheeses. Lait 78, 607 - 620).

1 litre of raw milk was pasteurised by heating for 30 minutes at 63 °C.

The pasteurised milk was then transferred into a wide mouthed plastic cheese vat (200 ml) and cooled down to 31°C.

Starter was added (2% in the case of fresh starter, Direct Set^{®} DS 5LT1 purchased by DSM Food Specialties Australia, 0.5 unit sachet 1.8 units/1,000L corresponding to 0.72 ml diluted starter/200ml) and incubated for 20 minutes.

132 µL CaCl₂ (1 M) was added followed by the addition of 43.5 µl of rennet (Maxiren^{®} 15 T purchased from DSM Food Specialties)

2 g of a formulation, according to Example 2, was added and the mixture was then stirred for 10 minutes.

After 50 minutes a firm coagulum was formed. The coagulum was cut manually by wire stretched 1 cm apart across a frame. Healing was allowed for 2 minutes followed by gently stirring for 10 minutes. The temperature was increased gradually to 38°C over 30 minutes and the curds/whey mixture stirred continuously. Upon reaching a pH of 6.2 the curds/whey mixtures were centrifuged at room temperature for 60 minutes at 1,700g. The whey was drained and the curds held in a water bath at 36°C. The cheeses were inverted every 15 minutes until the pH decreased to 5.2-5.3 and were then centrifuged at 1,700g for 20 minutes. After further whey drainage, the cheeses were brine salted (20% NaCl, 0.05% CaCl₂.H₂O) for 30 minutes at room temperature in bottles. After salting, the cheeses were removed from the cheese vats, wiped with tissue paper, vacuum packed and ripened at 12°C. A mass balance was established to control for the possible loss of enzyme activity during production. Samples were taken at every step in the cheese making process. The samples were stored at -20°C. A mass balance of the obtained samples is given in Table 3.

**Table 3. Mass balance cheese-making process**

| Mass Balance of cheese | Found enzyme activity % |
|---|---|
| After adding starter | 24 |
| After adding CaCl2 | 39 |
| After cutting | 35 |
| After increasing temperature | 23 |
| After first centrifuge step | 37 |
| Whey fraction | 0 |

Because the entrapment of the enzyme in the matrix not all the activity in the cheese/curd will be found.

## Claims

1. A matrix comprising:
- a hydrocolloid; and
- an enzyme in an amount from 0.01 to 20% w/w (based on dry matter of the matrix),
wherein the hydrocolloid is a protein, the matrix is in the form of particles and at least 50% of the matrix particles have a diameter of from 1 to 50 µm, the matrix has from 80 to 99.9% (w/w) dry matter content, and the enzyme is entrapped in the matrix.

2. A matrix according to claim 1 wherein the enzyme is soluble or partially soluble in water.

3. A matrix according to claim 1 or claim 2, wherein the hydrocolloid is selected from whey, a derivative of whey, casein, a derivative of casein, and a combination of a least two of these hydrocolloids.

4. A matrix according to claim 3, wherein the hydrocolloid is whey or a derivative of whey.

5. A matrix according to any one of claims 1 to 4, wherein the enzyme is a protease, a peptidase, an amino transferase, a lysase, a lipase or a combination thereof.

6. A method for the production of cheese, which comprises the addition of a matrix as defined in any one of claims 1 to 5, wherein the matrix is added prior to the separation of the curd from the whey.

7. Use of a matrix in the preparation of cheese, said matrix comprising:
- a hydrocolloid; and
- an active compound in an amount from 0.01 to 20% w/w (based on dry matter of the matrix),
wherein the hydrocolloid is a protein, the matrix is in the form of particles and at least 50% of the matrix particles have a diameter of from 1 to 50 µm, the matrix has from 80 to 99.9% (w/w) dry matter content, and the active compound is entrapped in the matrix.

8. Use according to claim 7, wherein the active compound is soluble or partially soluble in water.

9. Use according to claim 7 or claim 8, wherein the active compound is an enzyme.

10. Use according to claim 9, wherein the enzyme is a protease, a peptidase, an amino transferase, a lysase, a lipase or a combination thereof.

11. Use according to any one of claims 7 to 10, wherein the hydrocolloid is selected from whey, a derivative of whey, casein, a derivative of casein, and a combination of a least two of these hydrocolloids.

12. Use according to claim 11, wherein the hydrocolloid is whey or a derivative of whey.

## Patentansprüche

1. Matrix, umfassend:
- ein Hydrokolloid; und
- ein Enzym in einer Menge von 0,01 bis 20 Gew./Gew.-% (bezogen auf die Trockensubstanz der Matrix),
wobei das Hydrokolloid ein Protein ist, die Matrix in der Form von Teilchen vorliegt und wenigstens 50% der Matrixteilchen einen Durchmesser von 1 bis 50 µm aufweisen, die Matrix einen Trockensubstanzgehalt von 80 bis 99,9 Gew./Gew.-% aufweist und das Enzym in der Matrix gefangen ist.

2. Matrix gemäß Anspruch 1, wobei das Enzym in Wasser löslich oder teilweise löslich ist.

3. Matrix gemäß Anspruch 1 oder Anspruch 2, wobei das Hydrokolloid ausgewählt ist aus Molke, einem Molkederivat, Casein, einem Caseinderivat und einer Kombination wenigstens zwei dieser Hydrokolloide.

4. Matrix gemäß Anspruch 3, wobei das Hydrokolloid Molke oder ein Molkederivat ist.

5. Matrix gemäß einem der Ansprüche 1 bis 4, wobei das Enzym eine Protease, eine Peptidase, eine Aminotransferase, eine Lysase, eine Lipase oder eine Kombination davon ist.

6. Verfahren zum Herstellen von Käse, umfassend das Zugeben einer gemäß einem der Ansprüche 1 bis 5 definierten Matrix, wobei die Matrix vor dem Abtrennen des Bruchs von der Molke zugegeben wird.

7. Verwendung einer Matrix bei der Herstellung von Käse, wobei die Matrix Folgendes umfasst:
- ein Hydrokolloid; und
- einen Wirkstoff in einer Menge von 0,01 bis 20 Gew./Gew.-% (bezogen auf die Trockensubstanz der Matrix),
wobei das Hydrokolloid ein Protein ist, die Matrix in der Form von Teilchen vorliegt und wenigstens 50% der Matrixteilchen einen Durchmesser von 1 bis 50 µm aufweisen, die Matrix einen Trockensubstanzgehalt von 80 bis 99,9 Gew./Gew.-% aufweist und der Wirkstoff in der Matrix gefangen ist.

8. Verwendung gemäß Anspruch 7, wobei der Wirkstoff in Wasser löslich oder teilweise löslich ist.

9. Verwendung gemäß Anspruch 7 oder Anspruch 8, wobei der Wirkstoff ein Enzym ist.

10. Verwendung gemäß Anspruch 9, wobei das Enzym eine Protease, eine Peptidase, eine Aminotransferase, eine Lysase, eine Lipase oder eine Kombination davon ist.

11. Verwendung gemäß einem der Ansprüche 7 bis 10, wobei das Hydrokolloid ausgewählt ist aus Molke, einem Molkederivat, Casein, einem Caseinderivat und einer Kombination wenigstens zwei dieser Hydrokolloide.

12. Verwendung gemäß Anspruch 11, wobei das Hydrokolloid Molke oder ein Molkederivat ist.

## Revendications

1. Matrice comprenant :
- un hydrocolloïde ; et
- une enzyme en une quantité de 0,01 à 20 % p/p (par rapport à la matière sèche de la matrice)
dans laquelle l'hydrocolloïde est une protéine, la matrice est sous la forme de particules et au moins 50 % des particules de la matrice ont un diamètre de 1 à 50 µm, la matrice possède une teneur en matière sèche de 80 à 99,9 % (p/p) et l'enzyme est piégée dans la matrice.

2. Matrice selon la revendication 1, dans laquelle l'enzyme est soluble ou partiellement soluble dans l'eau.

3. Matrice selon la revendication 1 ou la revendication 2, dans laquelle l'hydrocolloïde est choisi parmi le lactosérum, un dérivé de lactosérum, la caséine, un dérivé de lactosérum et une combinaison d'au moins deux de ces hydrocolloïdes.

4. Matrice selon la revendication 3, dans laquelle l'hydrocolloïde est du lactosérum ou un dérivé de lactosérum.

5. Matrice selon l'une quelconque des revendications 1 à 4, dans laquelle l'enzyme est une protéase, une peptidase, une aminotransférase, une lysase, une lipase ou une combinaison de celles-ci.

6. Procédé de production de fromage, qui comprend l'ajout d'une matrice selon l'une quelconque des revendications 1 à 5, dans lequel la matrice est ajoutée avant que le caillé ne soit séparé du lactosérum.

7. Utilisation d'une matrice dans la préparation de fromage, ladite matrice comprenant :
- un hydrocolloïde ; et
- un composé actif en une quantité de 0,01 à 20 % p/p (par rapport à la matière sèche de la matrice),
dans laquelle l'hydrocolloïde est une protéine, la matrice est sous la forme de particules et au moins 50 % des particules de la matrice ont un diamètre de 1 à 50 µm, la matrice possède une teneur en matière sèche de 80 à 99,9 % (p/p) et le composé actif est piégé dans la matrice.

8. Utilisation selon la revendication 7, dans laquelle le composé actif est soluble ou partiellement soluble dans l'eau.

9. Utilisation selon la revendication 7 ou la revendication 8, dans laquelle le composé actif est une enzyme.

10. Utilisation selon la revendication 9, dans laquelle l'enzyme est une protéase, une peptidase, une aminotransférase, une lysase, une lipase ou une combinaison de celles-ci.

11. Utilisation selon l'une quelconque des revendications 7 à 10, dans laquelle l'hydrocolloïde est choisi parmi le lactosérum, un dérivé de lactosérum, la caséine, un dérivé de lactosérum et une combinaison d'au moins deux de ces hydrocolloïdes.

12. Utilisation selon la revendication 11, dans laquelle l'hydrocolloïde est du lactosérum ou un dérivé de lactosérum.
